# EUROPÄISCHE PATENTANMELDUNG

(11) **EP 2 311 858 A1**
(43) Veröffentlichungstag der Anmeldung: **20.04.2011**
(21) Anmeldenummer: 10161679.5
(22) Anmeldetag: 30.04.2010
(51) Int. Cl.: C07K 14/435

(54) **Verbesserte Fluoreszenzproteine, deren Herstellung und Verwendung**

(30) Priorität: 19.05.2009 DE 102009021990; 05.06.2009 DE 102009024281
(71) Anmelder: evocatal GmbH, 40225 Düsseldorf (DE)
(72) Erfinder: Eggert, Thorsten, Dr., 45259 Essen (DE); Hausmann, Sascha, Dr., 40591 Düsseldorf (DE); Puls, Michael, Dr., 50733 Köln (DE)
(74) Vertreter: Michalski Hüttermann & Partner Patentanwälte

(57) **Zusammenfassung**

Mit der vorliegenden Erfindung werden Varianten von Fluoreszenzproteinen angegeben, welche hinsichtlich ihrer Eigenschaften für den Einsatz als Reporterproteine und/oder in der Analytik verbessert sind. Insbesondere werden Varianten von Fluoreszenzproteinen angegeben, welche heller fluoreszieren, eine verbesserte Quantenausbeute zeigen und/oder verschobene Excitations- bzw. Emissionsspektren aufweisen. Die erfindungsgemäßen Fluoreszenzproteine weisen in ihrer LOV-Domäne neben dem Austausch eines Cystein gegen eine Aminosäure, die kein FMN kovalent bindet, wenigstens eine weitere Punktmutation auf.

## Beschreibung

Die vorliegende Erfindung betrifft verbesserte Fluoreszenzproteine, deren Herstellung und Verwendung.

Fluoreszenzproteine werden für die Bearbeitung verschiedener analytischer Fragestellungen genutzt. Neben dem sog. "green fluorescent protein" (GFP) und seinen Derivaten sowie anderen Fluoreszenzproteinen, deren Fluoreszenz auf die intrinsische Bildung eines Chromophors durch chemische Modifikation der in der Primärstruktur vorhandenen Aminosäuren beruht, wurden Fluoreszenzproteine beschrieben, die auf veränderten Blaulicht-Rezeptorproteinen basieren (Drepper *et al.,* 2007). Bei diesen in der Deutschen Patentanmeldung DE 10 2005 048 828 A1 beschriebenen Proteinen ist aufgrund einer Punktmutation der Photozyklus des Cofaktors Flavinmononukleotid (FMN), der in diesen Proteinen vorhanden ist, unterbrochen und die absorbierte Energie des eingestrahlten Lichts wird durch Fluoreszenz abgegeben. Die in den Fluoreszenzproteinen für die Fluoreszenz verantwortlichen Sequenzabschnitte werden als LOV-Domänen (Light, Oxygen, Voltage - Domänen) bezeichnet. Diese Form von Fluoreszenzproteinen wird von der Firma evocatal GmbH unter dem Produktnamen "evoglow®" vertrieben. Innerhalb der evoglow® - Produktreihe wird zwischen den aus unterschiedlichen Organismen gewonnenen Fluoreszenzproteinen unterschieden. So werden aus *Bacillus subtilis* gewonnene Fluoreszenzproteine als evoglow® -Bs bezeichnet und aus *Pseudomonas putida* gewonnene als evoglow® -Pp. Die Sequenzenprotokolle zu drei dieser Fluoreszenzproteine sind im Anhang angegeben und als Sequenz 1, 2 und 14 bezeichnet.

Fluoreszenzproteine können als sogenannte Reporterproteine in Zellen oder Organismen zur Beobachtung biochemischer Prozesse eingesetzt werden. Anwendungsbereiche sind dabei beispielsweise die Untersuchung genregulatorischer Mechanismen oder die Überwachung von biotechnologischen Prozessen. Es ist von Vorteil, Varianten der Fluoreszenzproteine zu nutzen, die hinsichtlich ihrer Helligkeit bzw. Quantenausbeute verbesserte Eigenschaften aufweisen, um auch schwache Fluoreszenzsignale der Proteine deutlich visualisieren zu können. Für die Anwendung der Fluoreszenzproteine in extremophilen Organismen müssen die Reporterproteine hinsichtlich der Stabilität gegen Denaturierung bei harschen Temperatur- und pH-Bedingungen angepasst werden, um ein Fluoreszenzsignal detektieren zu können. Weiterhin ist es bei bestimmten Untersuchungen wie z.B. bei Colokalisationsstudien von Proteinen notwendig, zwei verschiedene Populationen an Reporterproteinen in der gleichen Zelle visualisieren zu können. Dies wird vor allem durch unterschiedliche Excitations- und/oder Emissionseigenschaften (Farben) der gemeinsam genutzten Fluoreszenzproteine erreicht. Die bislang in der Literatur beschriebenen Fluoreszenzproteine weisen aufgrund ihres identischen Chromophors FMN alle ein vergleichbares Excitations- und Emissionsspktrum auf, wodurch eine Differenzierung der Proteine nicht möglich ist.

Es ist daher die **Aufgabe** der vorliegenden Erfindung, Varianten von Fluoreszenzproteinen zur Verfügung zu stellen, die hinsichtlich ihrer Eigenschaften für den Einsatz als Reporterprotein und/oder die Analytik verbessert sind. Dazu zählen zum Beispiel hellere Varianten mit verbesserter Quantenausbeute und/oder verschobenem Excitations- bzw. Emissionsspektrum.

**Gelöst** wird diese Aufgabe durch ein Fluoreszenzprotein mit einer LOV-Domäne, bei welcher mindestens ein Cystein durch eine andere Aminosäure, vorzugsweise Alanin, ersetzt ist, die kein FMN kovalent bindet, welches dadurch gekennzeichnet ist, dass die LOV-Domäne zur Verbesserung der Fluoreszenzintensität, der Photostabilität und/oder zur Veränderung der Fluoreszenzwellenlänge neben dem Austausch des mindestens einen Cystein wenigstens eine weitere Punktmutation aufweist.

In einer Ausführungsform der Erfindung weist das Fluoreszenzprotein eine LOV-Domäne auf, bei welcher mindestens ein Cystein durch eine andere Aminosäure, vorzugsweise Alanin, ersetzt ist, die kein FMN kovalent bindet, und welche neben dem Austausch des mindestens einem Cystein zwischen 1 und 30, vorzugsweise zwischen 1 und 20 und noch bevorzugter zwischen 1 und 10 weitere Punktmutationen aufweist.

In einer bevorzugten Ausführungsform der Erfindung ist die LOV-Domäne eine Aminosäuresequenz gemäß einer der Sequenzen 1, 2, 12 oder 14, welche wenigstens 1 Punktmutation, vorzugsweise zwischen 1 und 30, bevorzugter zwischen 1 und 20 und noch bevorzugter zwischen 1 und 10 Punktmutationen aufweist.

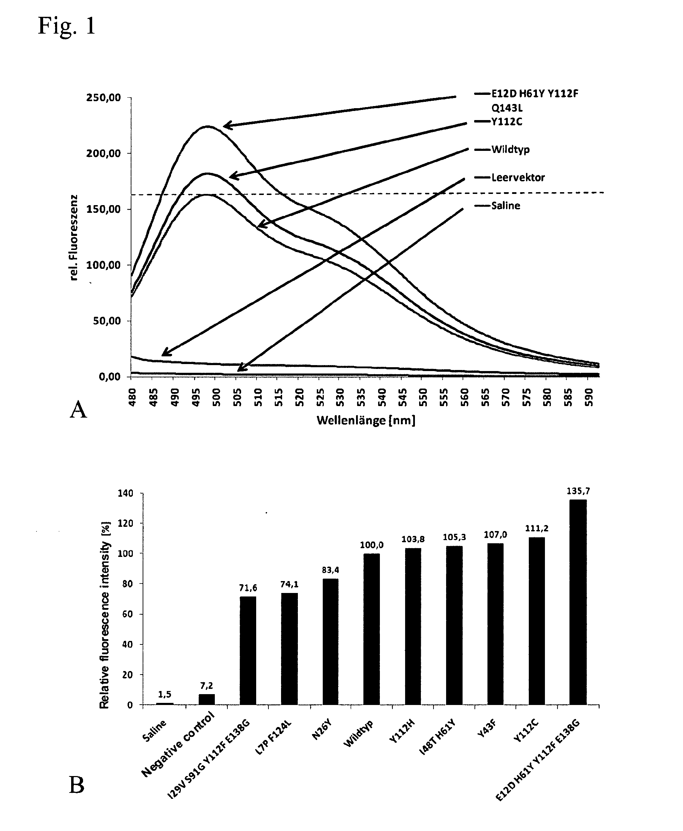

Zu diesem Zweck wurde durch gerichtete Evolution mit Hilfe von ep-PCR und Einbringung von zufälligen Mutationen in das Gen, welches für den Fluoreszenzmarker evoglow®-Pp1 codiert, eine Genbank erzeugt. Diese wurde im Folgenden in den Vektor pRhoKHi kloniert und in *E. coli* exprimiert, um die Varianten der Fluoreszenzproteine im Anschluss auf Veränderungen der Fluoreszenzeigenschaften untersuchen zu können. Durch dieses Vorgehen konnten verschiedene Positionen bzw. Mutationen identifiziert werden, die überraschenderweise eine Veränderung der Fluoreszenzintensität bedingen (siehe Fig. 1 und Tab. 1).

**Tabelle 1: Identifizierte Proteinvarianten des evoglow®-Pp1 und ihre Helligkeit. Die relative Intensität der Fluoreszenzemission wurde vergleichend mit dem Wildtyp-Protein einerseits anhand von Flüssigkulturen computergestützt mit der Bildanalysesoftware ImageJ und andererseits spektralphotometrisch im Fluoreszenzphotometer bestimmt. Die mit ImageJ bestimmten Daten stellen Mittelwerte aus zwei unabhängig durchgeführten Messungen dar.**

| | **rel. Intensität zu evoglow®-Pp1.** | |
|---|---|---|
| **Mutation** | **ImageJ** | **Fluoreszenzphotometer** |
| **Pp1 Wildtyp** | **100 ± 2** | **100 %** |
| **129V, S91G, Y112F, E138G** | **119 ± 2** | **71,6 %** |
| **L7P, F124L** | **100 ± 8** | **74,1 %** |
| **N26Y** | **119 ± 2** | **83,4 %** |
| **Y112H** | **134 ± 9** | **103,8 %** |
| **148T, H61Y** | **93 ± 2** | **105,3 %** |
| **Y43F** | **100 ± 7** | **107,0 %** |
| **Y112C** | **118 ± 12** | **111,2 %** |
| **E12D, H61Y, Y112F, Q143L** | **153 ± 1** | **137,1 %** |

Im Rahmen der Untersuchung der Banken konnten sowohl Varianten mit verringerten als auch erhöhten Fluoreszenzemissionen identifiziert werden. Bei dem Großteil dieser Varianten sind einige der Austausche in direkter räumlicher Nähe zum Cofaktor lokalisiert und/oder betreffen einen aromatischen Aminosäurerest, der durch die konjugierten Doppelbindungen beträchtliche Teile der eingestrahlten bzw. emittierten Strahlungsenergie absorbieren könnte (sog. Quenching-Effekt). Einige der mutagenisierten Positionen treten wiederholt auf, so dass sich sog. *hot-spots* für Mutationen ergeben, die in Tabelle 2 zusammengefasst sind.

**Tab. 2: Häufigkeit von Mutationen an ausgewählten Positionen im evoglow®-Pp1 mit nachgewiesenem Effekt auf die Helligkeit des evoglow®-Pp1.**

| **Positionen** | **Häufigkeit bei 10 Varianten** |
|---|---|
| **Y112H** | **4** |
| **H61Y** | **2** |

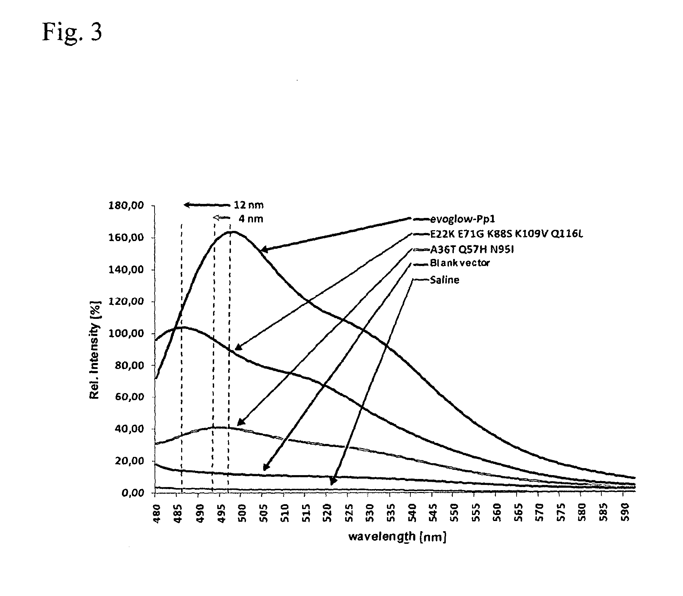

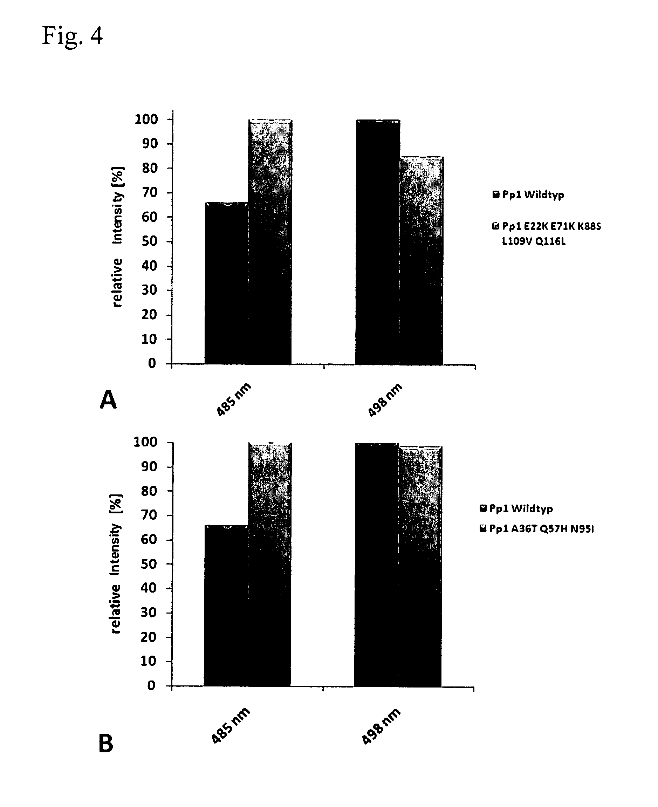

Desweiteren wurden zwei durch erfindungsgemäße Punktmutationen erhaltene Varianten des evoglow®-Pp1 mit in den kurzwelligeren Bereich verschobenem Emissionsspektrum identifiziert, die, verglichen mit dem Wildtyp-Protein, eher bläulich-grünes Licht emittieren (siehe Tab. 3 und Fig. 3 und 4).

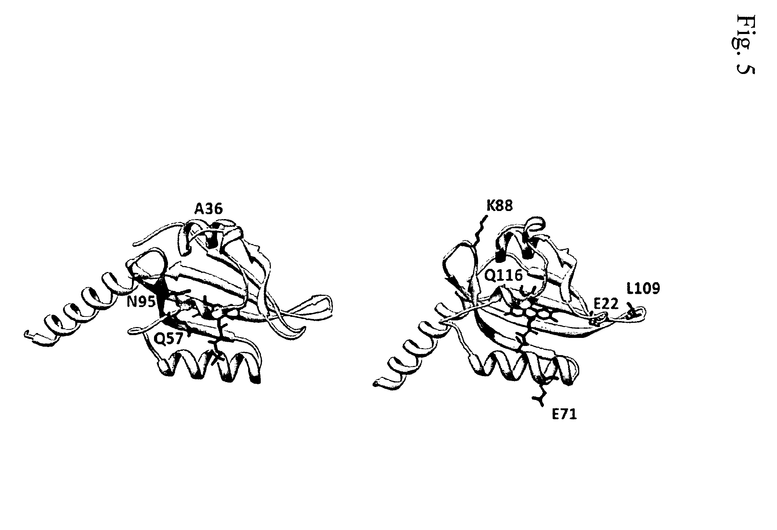

Die Verschiebung des Emissionsspektrums in den kurzwelligeren Bereich geht bei beiden Varianten mit einer Verringerung der Fluoreszenzintensität einher. Vermutlich liegt dies in der höheren Energie des emittierten Lichts mit einer geringeren Wellenlänge begründet. Eine Übersicht über die Mutationen, die relative Intensität der Fluoreszenz und Verschiebung des Emissionsmaximums gibt Tabelle 3. Die Lage der eingeführten Austausche im Strukturmodell des evoglow®-Pp1 ist in Abbildung 5 gezeigt.

**Tabelle 3: Identifizierte Proteinvarianten des evoglow®-Pp1 und ihre Helligkeit sowie die Verschiebung des Emissionsmaximums. Die relative Intensität der Fluoreszenzemission wurde vergleichend mit dem Wildtyp-Protein einerseits anhand von Flüssigkulturen computergestützt mit ImageJ und andererseits spektralphotometrisch im Fluoreszenzphotometer bestimmt. Die Emissionsmaxima wurden anhand der photometrisch erfassten Spektren ermittelt. Die mit ImageJ bestimmten Daten stellen Mittelwerte aus zwei unabhängig durchgeführten Messungen dar.**

| **Mutation** | **rel. Intensität zu evoglow®-Pp1** | | **Verschiebung des. Emissionsmaximums** |
|---|---|---|---|
| | **ImagJ** | **Fluoreszenzphotometer** | |
| **A36T, Q57H, N951** | **60,5 ± 7** | **24,7 %** | **- 4 nm (494 nm)** |
| **E22K, E71G, K88S, L109V, Q116L** | **109 ± 10** | **53,8 %** | **-12 nm (486 nm)** |

Durch die beiden eben beschriebenen Proteinvarianten konnte gezeigt werden, dass es prinzipiell möglich ist, die Emissionsspektren der evoglow®-Proteine und somit das farbliche Erscheinungsbild der Fluoreszenzreporter zu verändern. Diese Erkenntnis ist im Fall anderer Fluoreszenzreporter wie dem *green fluorescent protein* (GFP) und seiner Derivate relativ offensichtlich, da der Fluorophor durch Aminosäureseitenketten gebildet wird und somit eine Mutagenese dieser an der Reifung beteiligten Aminosäuren zur Generation neuer Farbvarianten genutzt werden kann. Im Fall der evoglow®-Proteine wird der Fluorophor jedoch nicht durch den Proteinanteil selbst, sondern durch die im Protein vorhandene sogenannte prosthetische Gruppe Flavinmononukleotid (FMN) gestellt. Dadurch wird das Fluoreszenzverhalten des Proteins im Gegensatz zu GFP in Bezug auf die Excitations- und Emissionseigenschaften hauptsächlich durch den Cofaktor und nicht durch den Proteinanteil selbst bestimmt. Aus diesem Grund ist es sehr erstaunlich, dass es möglich ist, die Fluoreszenzeigenschaften durch eine Veränderung des umgebenden Proteins, und nicht des Chromophors selbst, zu bewirken.

Beide Varianten mit verschobenem Fluoreszenzspektrum weisen eine oder mehrere Mutationen in direkter räumlicher Nähe zum Cofaktor auf. Hierbei gehen die mutagenisierten Aminosäurereste Q57, N95 und Q116 alle direkte ionische Bindungen mit dem Cofaktor ein und sind putativ an seiner korrekten Positionierung beteiligt. Ohne an diese Theorie gebunden zu sein wird derzeit davon ausgegangen, dass sich bei diesen Varianten durch eine leichte Verschiebung des Cofaktors in seiner Bindungskavität ein verändertes Fluoreszenzspektrum ergibt.

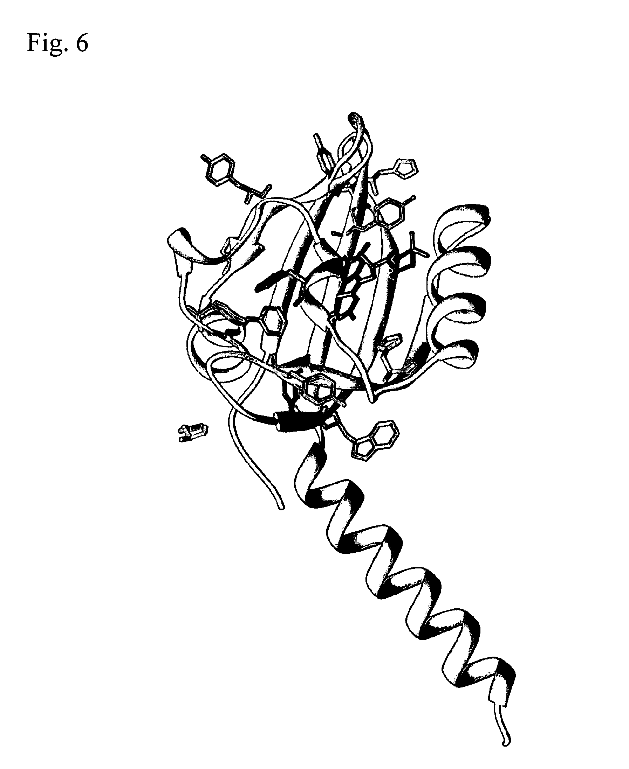

In Zusammenhang mit helleren Varianten des Fluoreszenzproteins wurden überraschend viele Austausche identifiziert, bei denen Aminosäuren mit Seitenketten betroffen waren, die in der Nähe des Chromophors lokalisiert sind und die aromatische Strukturen bzw. konjugierte Bindungen aufweisen. Aufgrund dieser Ergebnisse wurden weitere potentiell Einfluss nehmende Reste identifiziert, die durch ihre konjugierten π-Elektronen Energie in Form von Strahlung aufnehmen könnten und somit an Quenching-Reaktionen beteiligt sein können, die die Quantenausbeute und die Fluoreszenzintensität verringern. Eine Übersicht über diese Reste geben Figur 6 und Tabelle 4.

**Tab. 4: Übersicht über die im evoglow®-Pp1 enthaltenen Trp-, Tyr-, Phe- und His-Reste und ihre Entfernung zum FMN. Die Entfernung der Seitenketten wurde jeweils als kürzeste Entfernung zum FMN mit UCSF Chimera und dem Strukturmodell des evoglow®-Pp1 bestimmt.**

| **Rest** | **Entfernung zum FMN** **(kürzeste Distanz / Å)** |
|---|---|
| **Phe37** | **3,803** |
| **His61** | **3,835** |
| **Tyr112** | **3,897** |
| **Phe93** | **7,714** |
| **Phe55** | **7,995** |
| **Tyr86** | **8,141** |
| **Tyr111** | **9,508** |
| **Trp94** | **10,346** |
| **Tyr32** | **11,363** |
| **Tyr43** | **11,363** |
| **His103** | **14,647** |
| **Tyr50** | **14,929** |
| **His13** | **18,885** |

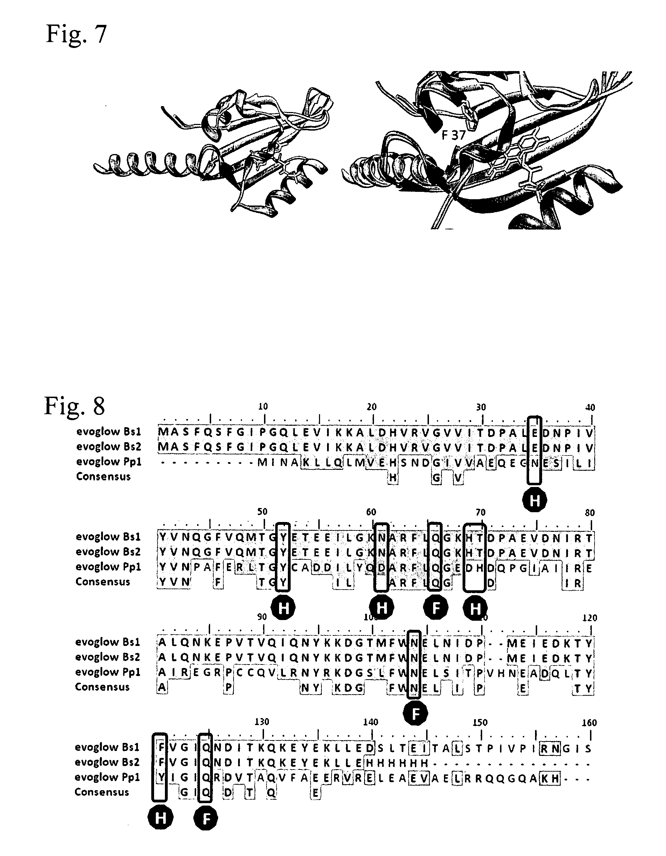

Es zeigt sich, dass eine Mutation der Reste His61 und Tyr112 eine Veränderung des Fluoreszenzverhaltens des Proteins bedingt (siehe Tab. 1 und Tab. 2). Im Fall des Phe37 fällt bei Analyse der Tertiärstrukturen von YtvA auf, dass eine deutliche lichtinduzierte Molekularbewegung im Dunkel- Hellübergang auftritt (Fig. 7). Somit stellt die Mutagenese dieses Restes ebenfalls eine Möglichkeit für die Bereitstellung neuer Fluoreszenzprotein-Varianten mit veränderten Helligkeiten und/oder Fluoreszenzspektren dar.

Ein vergleichendes Alignment der Primärstrukturen der evoglow®-Bs (*Bacillus subtilis*) Proteine mit der Aminosäuresequenz des evoglow®-Pp1 (*Pseudomonas putida*) liefert wichtige Daten hinsichtlich der Übertragbarkeit der gewonnenen Erkenntnisse auf andere FMN-basierte Fluoreszenzproteine (Fig. 8).

Einige der Varianten, die in Zusammenhang mit einer verringerten Fluoreszenzintensität gefunden wurden, trugen nur einen Austausch, der somit offensichtlich direkten Einfluss auf die Helligkeit nimmt. Auffällig ist, dass genau diese mutagenisierten Positionen (D52G bzw. N26Y) im Alignment nicht konserviert sind.

Wie bereits erwähnt, wurden im Rahmen der Mutagenese erstaunlich viele verbesserte Varianten identifiziert, bei denen ein Austausch von Aminosäuren stattgefunden hat, die eine direkte Interaktion mit dem Chromophor eingehen und in den evoglow® Proteinen gut konserviert sind. Somit ist es umso unerwarteter, dass gerade ein Austausch dieser als für die Funktion putativ essentieller Reste einen positiven Einfluss auf das Fluoreszenzverhalten ausüben kann.

Auf Basis dieser überraschenden Ergebnisse wurden anhand der aufgeklärten Struktur des YtvA Proteins weitere Aminosäuren identifiziert, die ebenfalls direkt eine Interaktion mit dem FMN eingehen und potentiell auf die Fluoreszenzeigenschaften der evoglow®-Proteine signifikanten Einfluss ausüben.

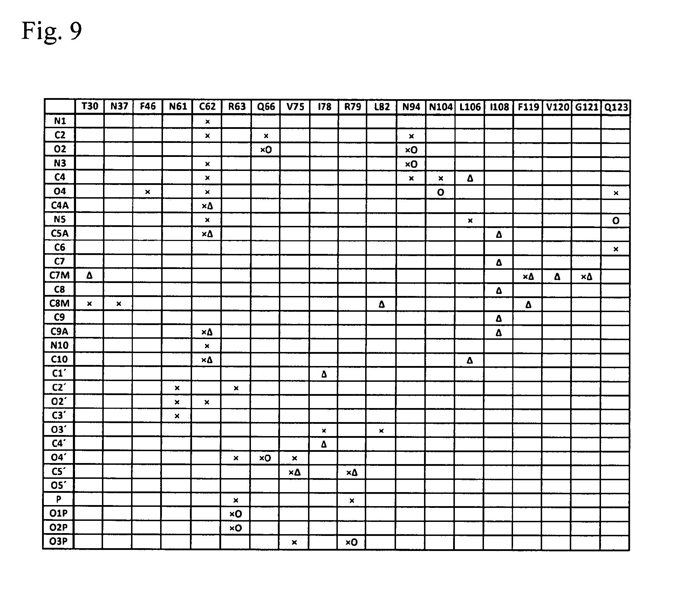

Eine Übersicht über die interagierenden Reste mit dem FMN und die Art der Wechselwirkung gibt Fig. 9.

Aus Fig. 9 und dem Alignment der evoglow®-Proteine (Fig. 8) ergeben sich folgende Positionen für die mit dem FMN interagierenden Aminosäuren in evoglow®- Bs1 bzw. -Bs2 sowie -Pp1 (siehe Tab. 5).

**Tab. 5: Übersicht über die mit dem FMN interagierenden Aminosäurereste im evoglow®-Bs1 bzw. Bs2 und dem evoglow®-Ppl. Die Positionen wurden basierend auf den Daten der Fig. 8 und 9 identifiziert.**

| **Art der interaktion** **(Δ: hydrophob;** ○**: hydrophil,** ×**: H Brücke)** | **Position in evoglow®-Bs1** **und evoglow®-Bs2** | **Homologe Position in evoglow®-Pp1** |
|---|---|---|
| Δ, × | **T30** | **A21** |
| × | **N37** | **N35** |
| × | **F46** | **F37** |
| × | **N61** | **D52** |
| Δ, × | **A62** | **A53** |
| ○, × | **R63** | **R54** |
| ○, × | **Q66** | **Q57** |
| Δ, × | **V75** | **I66** |
| Δ, ×, ○ | **I78** | **I69** |
| Δ, × | **R79** | **R70** |
| ○, × | **L82** | **I73** |
| ○, × | **N94** | **N85** |
| ○, × | **N104** | **N95** |
| Δ, × | **L106** | **L97** |
| Δ | **1108** | **I99** |
| Δ, × | **F119** | **Y112** |
| Δ | **V120** | **I113** |
| Δ, × | **G121** | **G114** |
| ○, × | **Q123** | **Q116** |

### Ausführungsbeispiele

### Ausführungsbeispiel 1) Generierung der Mutagenesebank der Gene, die für Fluoreszenzproteine codieren

Die Mutagenese der Gene, die für die Fluoreszenzreporter codieren, erfolgte durch error prone PCR. Hierzu wurden zufällige Punktmutationen durch die Nutzung der Taq-Polymerase und weiterer Additive wie MnCl₂ und unphysiologisch hoher Konzentrationen von MgCl₂ in das Wildtyp Gen eingeführt. Die Primer wurde so gewählt, dass sie das Gen flankierten und in die PCR-Produkte Erkennungssequenzen für die Restriktionsenzyme *Nde*I bzw. *Sac*l anfügten.

### Ausführungsbeispiel 2) Klonierung der Bank

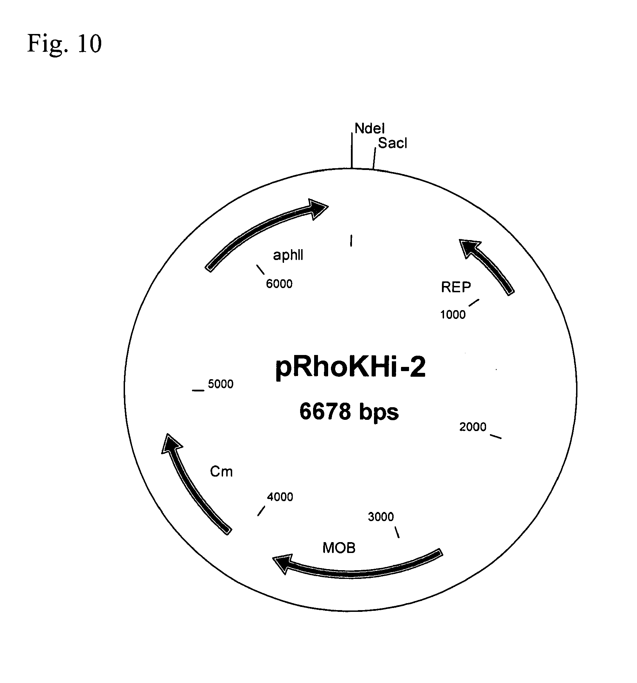

Die resultierenden ep-PCR Produkte wurden durch Ligation in den Vektor pRhoKHi-2 (Fig. 10) kloniert. Zu diesem Zweck wurden sowohl der Vektor als auch die PCR Produkte mit den Restriktionsendonukleasen Ndel und *Sac*l hydrolysiert. Nach der Inaktivierung der Enzyme wurden die Vektoren mit den PCR-Produkten ligiert und kompetente *E. coli* DH5α Zellen mit ihnen transformiert.

### Ausführungsbeispiel 3) Durchmusterung der Banken nach verbesserten Varianten

Das Screening nach verbesserten Proteinvarianten hinsichtlich ihrer Helligkeit wurde mit Hilfe eines UV-Kabinetts (Camag) durchgeführt. Die resultierenden Transformanden wurden direkt auf der LB-Agarplatte in der Blaulichtbox unter Anregung bei einer Wellenlänge von 366 nm mit einer Digitalkamera fotografiert. Die Analyse der Helligkeit der Kolonien auf der Platte erfolgte anhand dieser Bilder mit dem Programm ImageJ und dem Plugin "3D Surface Plot". Dieses Plugin erlaubt die Visualisierung von Helligkeiten eines Bildes als graphische Darstellung von Höhentopologien.

Auch die Bestimmung der Farbe der Kulturen erfolgte nach Dokumentation der Kolonien mit einer Digitalkamera computergestützt mit dem Programm ImageJ, Zu diesem Zweck wurde das Plugin "3D Color Inspector" verwendet, welches erlaubt, Farbverteilungen eines Bildes im dreidimensionalen Raum zu visualisieren.

### Ausführungsbeispiel 4) Messung der Fluoreszenz von Flüssigkulturen

Potentiell hellere Klone wurden zusammen mit einer Kultur, die ein Plasmid mit dem nicht mutagenisierten Fluoreszenzreporter trug, erneut als Flüssigkultur angezogen und über Nacht bei 37 °C und Schüttlergeschwindigkeiten von 180 Upm bebrütet. Danach wurde die O.D._{580 nm} der Kulturen bestimmt und die Zellen durch Zentrifugation bei 8000g für 5 min sedimentiert. Das resultierende Zellpellet wurde einmal mit Saline gewaschen und anschließend in einem Volumen Saline resuspendiert, so dass eine O.D._{580 nm} von 25 erreicht wurde. Jeweils 10 µL dieser Proben sowie eine Verdünnungsreihe in 1 zu 10 Verdünnungen wurden auf eine Plexiglasplatte überführt und mit Hilfe der Blaulichtbox und einer Digitalkamera dokumentiert. Die Auswertung der Bilder erfolgte wie zuvor beschrieben mit ImageJ und dem "3D Surface Plot" Plugin, wobei die Kultur, die den nicht mutagenisierten Fluoreszenzreporter exprimierte, als Referenz genutzt wurde.

### Ausführungsbeispiel 5) Aufnahme von Fluoreszenzspektren von Flüssigkulturen

Fluoreszenzmessungen ganzer Zellen wurden in 100 mM Tris-HCl pH 7.0 durchgeführt. Zu diesem Zweck wurden O.D._{580 nm} = 1 entsprechende Aliquots den jeweiligen Expressionskulturen entnommen, pelletiert (3 min, 8000 UpM, Raumtemperatur) und mit 100 mM Tris-HCl pH 7.0 gewaschen. Anschließend wurde die Zellsuspension mit Saline auf eine Zelldichte eingestellt, die einer 0.D._{580 nm} von 0,5 entspricht. Die Fluoreszenz der Proben wurde mit einem Fluoreszenzphotometer (Luminescence Spectrometer LS 50B, Perkin Elmer, Boston, USA) gemessen.

### Figurenbeschreibung:

Fig. 1**:** (A) Emissions-Fluoreszenzspektren von *E*. *coli* Flüssigkulturen, die den Fluoreszenzmarker evoglow®-Pp1 bzw. ausgewählte mutagenisierte Varianten exprimieren. Die Zellen wurden mit Saline gewaschen und auf eine Zelldichte normiert, die einer O.D._{580 nm} von 0,5 entspricht. Das Emissionsspektrum wurde bei einer Anregungswellenlänge von 495 nm aufgezeichnet. (B) Zusammenfassung der Veränderung der relativen Fluoreszenzintensität am absoluten Maximum. Die Daten wurden den aufgezeichneten Fluoreszenzspektren entnommen.

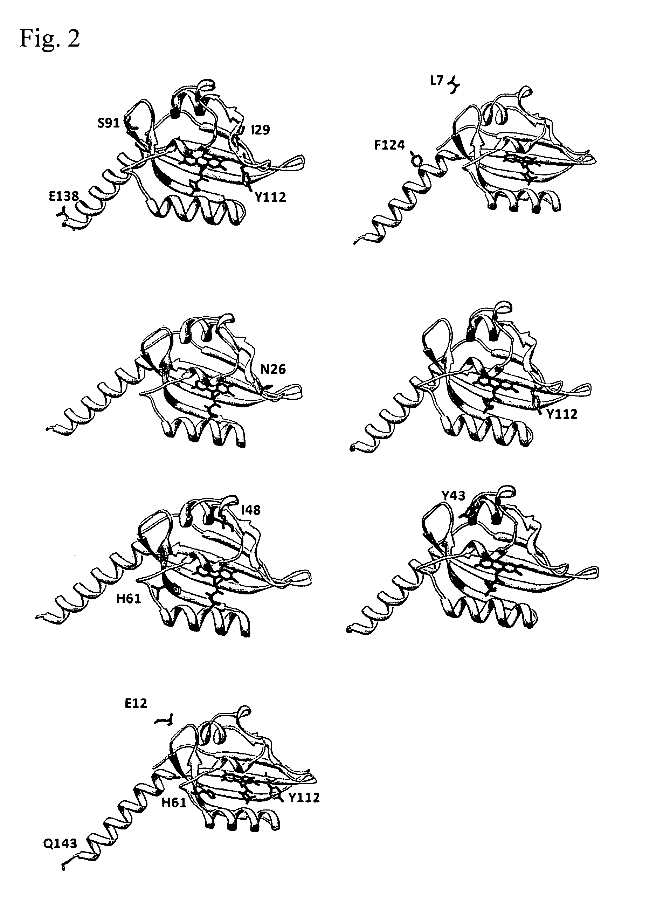

**Fig. 2****:** Lage der mutagenisierten Aminosäuren im Strukturmodell des evoglow®-Pp1 in Varianten, bei denen eine Veränderung der Helligkeit nachgewiesen wurde. In dunklerem Grau gezeigte Aminosäuren zeigen entsprechende Positionen mit identifizierten Austauschen an, wobei einige Reste der homologen Struktur von YtvA entnommen sind, da sie bei der Modellgenerierung von Pp1 nicht mit berechnet wurden. Im Fall des L7 wurde die erste in den Datensätzen enthaltene Aminosäure (V11) hervorgehoben. Die Strukturen wurden mit UCSF Chimera visualisiert.
**Fig. 3****:** Emissions-Fluoreszenzspektren von Kulturen, die evoglow®-Pp1 und identifizierte Varianten mit unterschiedlichen Emissionsmaxima exprimieren. Die Kulturen wurden mit Saline gewaschen und auf eine Zelldichte eingestellt, die einer O.D._{580 nm} von 0,5 entspricht.
**Fig. 4****:** Gegenüberstellung der relativen Intensitäten der Pp1 Varianten mit verschobenen Emissionsspektren gegen die des Wildtyps bei den jeweiligen absoluten Maxima.
**Fig. 5****:** Lage der mutagenisierten Aminosäuren im Strukturmodell des evoglow®-Pp1 in Varianten, bei denen eine Farbverschiebung gezeigt werden konnte. In Dunkelgrau hervorgehobene Aminosäuren zeigen entsprechende Positionen mit identifizierten Austauschen an. Die Strukturen wurden mit UCSF Chimera visualisiert.
**Fig. 6****:** Darstellung aller Positionen im evogiow®-Pp1 Strukturmodell, an denen Trp, Tyr, Phe oder His vorliegt, Der Cofaktor FMN ist zentral in Dunkelgrau gezeigt. Die Struktur wurde mit UCSF Chimera visualisiert.
**Fig. 7****:** Dunkel- und Hellstrukturen von YtvA aus *B. subtilis* (PDB Codes: 2PR5 bzw. 2PR6). Die bei Dunkelheit aufgeklärte Struktur ist in hellgrau dargestellt, diejenige unter Beleuchtung in cyan. Die Proteine sind in der *ribbon*-Darstellung gezeigt. Der Cofaktor FMN sowie der dem F37 entsprechende Rest F46 des YtvA sind im stick-Modus gezeigt.
**Fig. 8****:** Alignment der Primärsequenzen der Fluoreszenzproteine evoglow®-BsX und evoglow®-Pp1. Das Alignment wurde mit dem ClustalW Algorithmus durchgeführt und mit BioEdit dargestellt. Die in blau umrahmten und mit F gekennzeichneten Reste kennzeichnen Positionen, die bislang in Zusammenhang mit Farbvariation der Proteine gefunden und diese putativ bedingen. Die rot umrahmten Aminosäuren zeigen Positionen, die wahrscheinlich in Zusammenhang mit der Änderung der Fluoreszenzintensität stehen.
**Fig. 9****:** lnteraktionskarte des Proteinanteils des YtvA mit dem Cofaktor FMN. Die beteiligten Atome des Cofaktors sind vertikal, die Aminosäurereste des Proteins horizontal angegeben. Die Art der Interaktion ist durch folgende Symbole angezeigt: hydrophobe Interaktion: Δ; hydrophile Interaktion: ○; H-Brücke: ×.
**Fig. 10****:** Vektorkarte von pRhoKHi-2

## Patentansprüche

1. Fluoreszenzprotein mit einer LOV-Domäne, bei welcher mindestens ein Cystein durch eine andere Aminosäure, vorzugsweise Alanin, ersetzt ist, die kein FMN kovalent bindet, **dadurch gekennzeichnet, dass** die LOV-Domäne zur Verbesserung der Fluoreszenzintensität, der Photostabilität und/oder zur Veränderung der Fluoreszenzwellenlänge neben dem Austausch des mindestens einen Cystein wenigstens eine weitere Punktmutation aufweist.

2. Fluoreszenzprotein gemäß Anspruch 1, wobei die LOV-Domäne eine Aminosäuresequenz gemäß Sequenz 1, 2, 12 oder 14 ist, welche wenigstens eine weitere Punktmutation aufweist.

3. Fluoreszenzprotein gemäß Anspruch 2, wobei die LOV-Domäne wenigstens eine Punktmutation der Gruppe bestehend aus 129V, S91G, Y112F, E138G, L7P, F124L, N26Y, Y112H, I48T, H61Y, Y43F, Y112C, E12D, Q143L, A36T, Q57H, N951, E22K, E71G, K88S, L109V und Q116L aufweist.

4. Fluoreszenzprotein gemäß einem der Ansprüche 1 bis 2, aufweisend ein gegenüber einem Fluoreszenzprotein mit einer LOV-Domäne bei welcher mindestens ein Cystein durch eine andere Aminosäure, vorzugsweise Alanin, ersetzt ist, die kein FMN kovalent bindet, um wenigstens 4 nm, vorzugsweise wenigstens 10 nm verschobenen Emissionsmaximum.

5. LOV-Domäne, bei welcher mindestens ein Cystein durch eine andere Aminosäure, vorzugsweise Alanin, ersetzt ist, die kein FMN kovalent bindet, **dadurch gekennzeichnet, dass** diese zur Verbesserung der Fluoreszenzintensität, der Photostabilität und/oder zur Veränderung der Fluoreszenzwellenlänge neben dem Austausch des mindestens einen Cystein wenigstens eine weitere Punktmutationen aufweist.

6. LOV-Domäne gemäß Anspruch 5, wobei die LOV-Domäne eine Aminosäuresequenz gemäß Sequenz 1, 2, 12 oder 14 ist, welche wenigstens eine weitere Punktmutation aufweist.

7. LOV-Domäne gemäß Anspruch 6, wobei diese wenigstens eine Punktmutation der Gruppe bestehend aus I29V, 891G, Y132F, E138G, L7P, F124L, N26Y, Y112H, 148T, H61Y, Y43F, Y112C, E12D, Q143L, A36T, Q57H, N95I, E22K, E71G, K88S, L109V und Q116L aufweist.

8. LOV-Domäne gemäß Anspruch 6, wobei diese eine Nucleinsäuresequenz gemäß einer der Sequenzen 16 bis 25 ist.

9. Ribonucleinsäure, **dadurch gekennzeichnet, dass** sie für ein Fluoreszenzprotein gemäß einem der Ansprüche 1 bis 4 oder eine LOV-Domäne gemäß einem der Ansprüche 5 bis 8 kodiert.

10. Verfahren zur Herstellung eines Fluoreszenzproteins, wobei ein Plasmid, welches eine Ribonucleinsäure gemäß Anspruch 9 aufweist mittels gentechnische Methoden in einen Organismus, vorzugsweise ein Bakterium ausgewählt aus der Gruppe bestehend aus Escherichia coli, Rhodobacter capsulatus, Pseudomonas putida und Bacillus subtilis, eingebracht und dort exprimiert wird.

11. Verwendung eines Fluoreszenzproteins gemäß einem der Ansprüche 1 bis 4 als Fluoreszenzmarker, insbesondere in Lösungen oder Zellen.

## Geänderte Patentansprüche

### Geänderte Patentansprüche gemäss Regel 137(2) EPÜ.

**1.** Fluoreszenzprotein mit einer LOV-Domäne, bei welcher mindestens ein Cystein durch eine andere Aminosäure, vorzugsweise Alanin, ersetzt ist, die kein FMN kovalent bindet, **dadurch gekennzeichnet, dass** die LOV-Domäne zur Verbesserung der Photostabilität und/oder zur Veränderung der Fluoreszenzwellenlänge neben dem Austausch des mindestens einen Cystein wenigstens eine weitere Punktmutation aufweist.

**2.** Fluoreszenzprotein gemäß Anspruch 1, wobei die LOV-Domäne eine Aminosäuresequenz gemäß Sequenz 1, 2, 12 oder 14 ist, welche wenigstens eine weitere Punktmutation aufweist

**3.** Fluoreszenzprotein gemäß Anspruch 2, wobei die LOV-Domäne wenigstens eine Punktmutation der Gruppe bestehend aus 129V, S91G, Y112F, E138G, L7P, F124L, N26Y, Y112H, I48T, H61Y, Y43F, Y112C, E12D, Q143L, A36T, Q57H, N951, E22K, E71G, K88S, L109Vund Q116L aufweist.

**4.** Fluoreszenzprotein gemäß einem der Ansprüche 1 bis 2, aufweisend ein gegenüber einem Fluoreszenzprotein mit einer LOV-Domäne bei welcher mindestens ein Cystein durch eine andere Aminosäure vorzugsweise Alanin, ersetzt ist, die kein FMN kovalent bindet, um wenigstens 4 nm, vorzugsweise wenigstens 10 nm verschobenen Emissionsmaximum,

**5.** LOV-Domäne, bei welcher mindestens ein Cystein durch eine andere Aminosäure, vorzugsweise Alanin, ersetzt ist, die kein FMN kovalent bindet, **dadurch gekennzeichnet, dass** diese zur Verbesserung der Photostabilität und/oder zur Veränderung der Fluoreszenzwellenlänge neben dem Austausch des mindestens einen Cystein wenigstens eine weitere Punktmutation aufweist.

**6.** LOV-Domäne gemäß Anspruch 5, wobei die LOV-Domäne eine Aminosäuresequenz gemäß Sequenz 1, 2, 12 oder 14 ist, welche wenigstens eine weitere Punktmutation aufweist

**7.** LOV-Domäne gemäß Anspruch 6, wobei diese wenigstens eine Punktmutation der Gruppe bestehend aus I29V, S91G, Y112F, E138G, L7P, F124L, N26Y, Y112H, I48T, H61Y, Y43F, Y112C, E12D, Q143L, A36T, Q57H, N951, E22K, E71G, K88S, L109V und Q116L aufweist.

**8.** LOV-Domäne gemäß Anspruch 6, wobei diese eine Nucleinsäuresequenz gemäß einer der Sequenzen 16 bis 25 ist.

**9.** Ribonucleinsäure, **dadurch gekennzeichnet, dass** sie für ein Fluoreszenzprotein gemäß einem der Ansprüche 1 bis 4 oder eine LOV-Domäne gemäß einem der Ansprüche 5 bis 8 kodiert.

**10.** Verfahren zur Herstellung eines Fluoreszenzproteins, wobei ein Plasmid, welches eine Ribonucleinsäure gemäß Anspruch 9 aufweist mittels gentechnischer Methoden in einen Organismus, vorzugsweise ein Bakterium ausgewählt aus der Gruppe bestehend aus Escherichia coli, Rhodobacter capsulatus, Pseudomonas putida und Bacillus subtilis, eingebracht und dort exprimiert wird.

**11.** Verwendung eines Fluoreszenzproteins gemäß einem der Ansprüche 1 bis 4 als Fluoreszenzmarker, insbesondere in Lösungen oder Zellen.
